## Europäisches Patentamt
## European Patent Office
## Office européen des brevets

(19)

(11) Veröffentlichungsnummer : **0 219 488**
**B1**

(12)

# EUROPÄISCHE PATENTSCHRIFT

(45) Veröffentlichungstag der Patentschrift :
**03.01.90**

(21) Anmeldenummer : **86890277.6**

(22) Anmeldetag : **09.10.86**

(51) Int. Cl.$^5$ : **A 23 K   1/00, A 23 K   3/03,
C 12 N   1/20, C 12 R   1/46**

(54) Verwendung eines neuen Stammes von Streptococcus faecium Bakterien zur Absenkung des pH-Wertes im Magen-Darm-Trakt von Tieren, sowie der neue Stamm selbst.

(30) Priorität : 11.10.85 AT 2942/85

(43) Veröffentlichungstag der Anmeldung :
**22.04.87 Patentblatt 87/17**

(45) Bekanntmachung des Hinweises auf die Patenterteilung : **03.01.90 Patentblatt 90/01**

(84) Benannte Vertragsstaaten :
**AT BE CH DE FR GB IT LI LU NL SE**

(56) Entgegenhaltungen :
**DE--A-- 2 010 654
DE--A-- 2 803 794
LU--A--    60 508**

(73) Patentinhaber : **MIRIMI Erzeugungs-, Handels- und
Vertriebsgesellschaft m.b.H.
Kremser Landstrasse 5
A-3100 St. Pölten (AT)**

(72) Erfinder : **Thoma, Karl
Ratzersdorfer Hauptstrasse 120
A-3100 St. Pölten (AT)**

(74) Vertreter : **Itze, Peter, Dipl.-Ing. et al
Patentanwälte Dipl.-Ing. Leopold Friebel Dipl.-Ing.
Peter Itze Amerlingstrasse 8
A-1060 Wien (AT)**

## Beschreibung

Die Erfindung bezieht sich auf eine Verwendung eines neuen Stammes von Streptococcus faecium Bakterien zur Absenkung des ph-Wertes im Magen-Darm-Trakt von Tieren, sowie auf den neuen Stamm selbst.

Der Zusatz von lebenden, Milchsäure produzierenden Keimen, speziell der Gattung Streptococcus und/oder Lactobacillus, zu Futtermitteln soll durch Absenkung des pH-Wertes im Magen-Darm-Trakt während der Verdauung die Entwicklung von acidophoben und gegebenenfalls enteropathogenen Escherichiia coli-Keimen und verwandten Arten verhindern helfen.

Insbesondere bieten sich dazu Stämme aus der Gruppe der Enterococcen, speziell von Streptococcus (im folgenden mit Sc. abgekürzt) faecium, Sc. durans und Sc. faecium/durans an, da diese Keime ein Bestandteil der physiologischen Darmflora von Mensch und Tier sind und als homofermentative Milchsäurebakterien zu kräftiger Säurebildung befähigt sind. Darüberhinaus zeichnen sich diese Keime durch eine verhältnismäßig hohe Resistenz gegenüber diversen Umwelteinflüssen aus.

Naturgemäß gibt es auch innerhalb der Art Sc. faecium oder Sc. durans art- und stammspezifische Unterschiede, zumindest in den nachfolgend aufgezeigten Kriterien :

Effektive Wachstumgeschwindigkeit (Generationszeit),

Effektive Säureproduktion (Säuerungsaktivität), Relative Hitzeresistenz (Überleben technologischer Erhitzungs- und Trocknungsbedingungen),

Stabilität bei geringer Wasseraktivität (Erhalt der Lebendkeimzahl im getrockneten Futtermittel).

Zur Erzielung der gewünschten physiologischen Wirkung sollen die Bakterien eine kurze Generationszeit, eine hohe Säuerungsaktivität, eine relativ hohe Hitzeresistenz und eine hohe Stabilität bei geringer Wasseraktivität aufweisen.

Erfindungsgemäß werden zur Erzielung der gewünschten physiologischen Wirkungen Bakterien mit den identifizierenden Merkmalen des Stammes Sc. faecium IMB 52 (hinterlegt unter Nummer DSM 3530) eingesetzt. Dieser Stamm erbringt eine sehr rasche Absenkung des pH-Wertes, wodurch ein sehr schnell vor sich gehender Eiweißabbau erzielt wird, was eine bessere Verdauung und damit eine Erhöhung des allgemeinen Wohlbefindens der Tiere ergibt. Außerdem wird eine Säuerung auf einen pH-Wert erzielt, der um etwa 0,5 tiefer liegt, als bei bekannten Vergleichsstämmen. Schließlich bewirkt der angeführte Stamm einen raschen Wiederaufbau der Darmflora, z. B. bei höherer Antibiotikagabe, so daß es angezeigt ist, bereits während der Behandlung mit Antibiotika Sc. faecium IMB 52 in entsprechender Keimzahl zu verabreichen.

Vorteilhafterweise können die Bakterien dem Futter unter Vorsäuerung desselben beigegeben werden, wodurch die Bakterien bereits voll aktiviert sind, wenn sie in den Körper gelangen. Dabei können die Bakterien dem Futter zur Erzeugung von Silagefutter mit hohem Säuerungsgrad beigegeben werden.

Auf Grund des Säuerungsverhaltens können Bakterien mit den identifizierenden Merkmalen des Stammes Sc. faecium IMB 52 als mikrobielle Leistungsförderer verwendet werden. Unter Leistungsförderern werden Futterzusatzstoffe verstanden, welche eine höhere Fleischleistung der Schlachttiere in bezug auf die eingesetzte Futtermenge und Fütterungszeit bewirken.

Der erfindungsgemäße Mikroorganismus, der durch die identifizierenden Merkmale des Stammes Sc. faecium IMB 52 gekennzeichnet ist, eignet sich daher insbesondere zur Regeneration der Darmflora von Tieren, bzw. zur Absenkung des pH-Wertes im Magen-Darm-Trakt von Tieren, bzw. zur Säuerung von pflanzlichen Nahrungs- und Futtermitteln.

Die identifizierenden Merkmale, also die wissenschaftlichen Kennzeichen, des Stammes Sc. faecium IMB 52 sind folgende :

1. Mikroskopisches Bild (MRS-Bouillon, 24 Stunden, 35 °C) : grampositive Coccen, $\varnothing$ 1 $\mu$m, vorwiegend Diploform bis Kurzketten.

2. Wachstum positiv (in nicht selektiven Medien) : 10 °C, 45 °C in Gegenwart von 40 % Galle und in Gegenwart von 6,5 % NaCl.

Relativ unempfindlich gegenüber Zinkbacitracin, Virginiamycin und Flavomycin, hingegen relativ empfindlich gegenüber Penicillin.

3. Serologischer Test : D-Streptococcus.

4. Generationszeit (unter Optimalbedingungen) : 18 Minuten.

5. pH-Minimum (in Magermilch) : pH 4,55.

6. Zuckervergärung (« Bunte Reihe ») : Abweichend von anderen Sc. faecium-Stämmen ist IMB 52 negativ bei Saccharosevergärung (Übereinstimmung mit Sc. durans) und auch negativ im Melibiosetest.

7. Elektrophoretisches Proteinbandenmuster : Charakteristische Proteinbandenbereiche in Übereinstimmung mit anderen Sc. faecium-Stämmen, aber auch typische Differenzen bei Einzelbandenpositionen.

8. Stabilität : Relativ resistent gegenüber Hitzeeinfluß und stabil bei Lagerung in Trockenmilch während mindestens 6 Monaten.

Nachstehend wird anhand der angeschlossenen Diagramme und Tabellen die Isolation und die Wirkung des erfindungsgemäßen Bakterienstammes beschrieben. Fig. 1 ist eine schematische Darstellung des Identifikationsvorganges für Sc. faecium/durans. Fig. 2 gibt das elektrophoretische Trennbild intrazellulärer Proteine von Sc. faecium-Isolaten und Referenzstämmen wieder. Fig. 3 veranschaulicht die Wachstumskurven von Sc. faecium-Isolaten und Referenzstämmen. Fig. 4 zeigt die Säuerungsvermögen bei Anzucht von Sc. faeciun-Isolaten und Referenzstämmen in rekonstituierter Trockenmagermilch bei 35 °C. Fig. 5 ist eine graphische Darstellung der Veränderung der Lebendkeimzahl (KbE) während der Lagerung von sprühgetrockneten Magermilchkulturen von Sc. faecium-Isolaten sowie Referenzstämmen bei Zimmertemperatur. Fig. 6 zeigt Wachstums-Trübungskurven von Sc. faecium IMB 52. Fig. 7 veranschaulicht die Säuerungsaktivitätskurve von Sc. faecium IMB und einem Referenzstamm.

Es wurde von fünfunddreißig Isolaten von Sc. faecium aus dem Darminhalt von Schlachtkälbern ausgegangen, die vergleichend auf die angestrebten Eigenschaften geprüft wurden. Als Vergleich wurden zwei Sc. faecium-Stämme, die als Futtermittelzusatz bereits Verwendung finden, in diesen Test eingeschlossen. Es handelt sich dabei um das LBC-Lactobacterienkonzentrat Sc. faecium Cernelle 68 (im folgenden SF 68 genannt) und um Lactiferm Sc. faecium M 74 (im folgenden M 74 genannt).

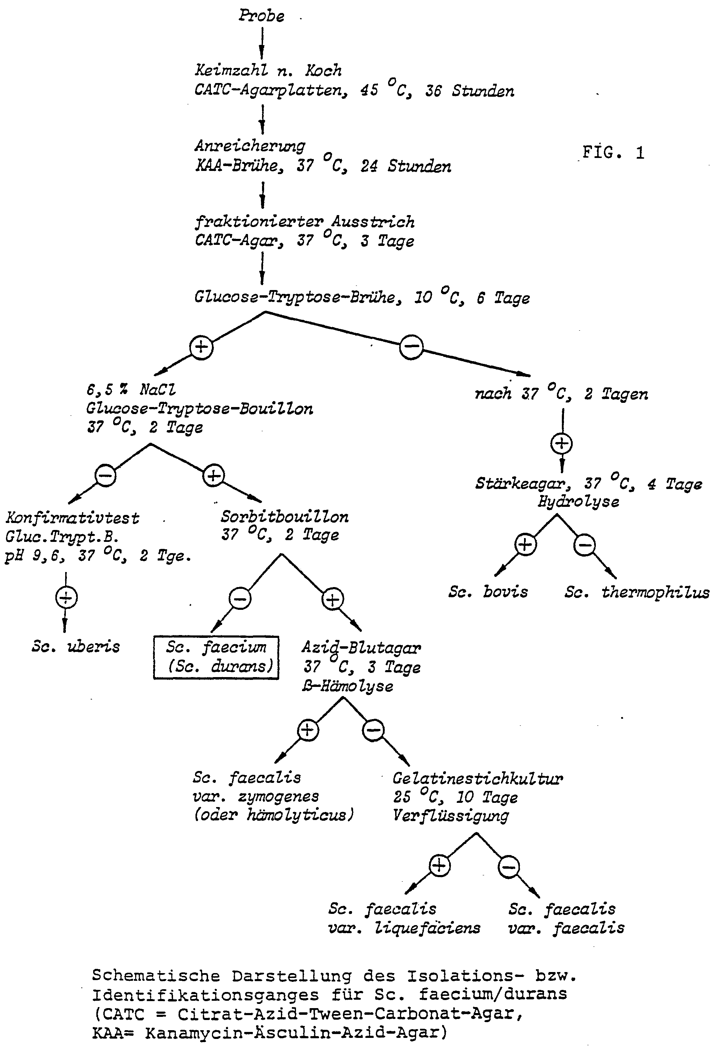

Die Identifikation von Sc. faecium/durans wird gemäß dem in Fig. 1 dargestellten Schema durchgeführt, wobei als wichtigstes Kriterium zur Eingrenzung von Sc. faecium-Stämmen nach selektiver Anreicherung der Enterococcen auf flüssigen und festen Nährmedien das Vergärungsvermögen von Sorbit, Arabinose und Mannit herangezogen wird. Wie aus Tabelle 1 ersichtlich, werden auf Grund dieser Vorgangsweise zunächst aus 75 Enterococcen-Isolaten 20 Sc. faecalis-Stämme, 20 Sc. faecium-Stämme, 12 Sc. faecium/durans-Stämme und 3 Sc. durans-Stämme identifiziert. Die weitere Charakterisierung der Isolate erfolgt mittels eines Zuckerverwertungsspektrums, der sogenannten « Bunten Reihe », elektrophoretischer Trennung von intrazellulären Proteinen, Resistenzverhalten gegenüber ausgewählten Antibiotika, Wachstumsgeschwindigkeitstests und schließlich Hitzestabilitätstests.

Bei der Darstellung der Ergebnisse in den angeschlossenen Figuren und Tabellen wird bereits vorweggenommen, daß drei der insgesamt geprüften Stämme (u. zw. IME 12, IMB 49 und IMB 52) als dem gesteckten Ziel besonders entsprechend erkannt werden.

In Tabelle 2 ist das Zuckerverwertungsvermögen der genannten Stämme den beiden Referenzstämmen Sf 68 und M 74 gegenübergestellt. Während sich die Stämme IMB 12 und IMB 49 identisch zu den untereinander ebenfalls identischen Referenzstämmen verhalten, zeichnet sich der Stamm Sc. faecium IMB 52 durch fehlende Saccharose- und Melitiosevergärung aus.

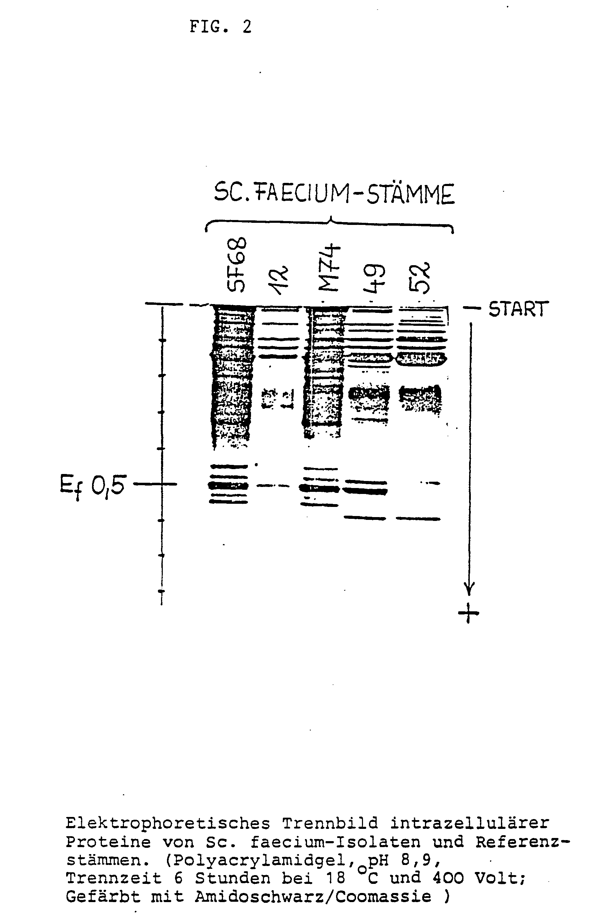

Bei der elektrophoretischen Trennung intrazellulärer Proteine nach völlig einheitlicher Anzucht und Aufarbeitung der Stämme wird die weitgehende Identität von SF 68 und M 74 noch deutlicher ersichtlich (s. dazu Fig. 2). Auch der Stamm IMB 12 hat große Ähnlichkeit zu den beiden vorgenannten Stämmen, wogegen der Stamm IMB 49 sich vor allem im Bereich E 0,5 und der Stamm IMB 52 sowohl im Bereich $E_f$ 0,1 als auch $E_f$ 0,5 deutlicher von den Referenzstämmen unterscheidet.

scheidet.

In Tabelle 3 ist die Resistenz bzw. die Empfindlichkeit ausgewählter Stämme und der Referenzstämme gegenüber einigen Antibiotika wiedergegeben. Die Konzentration von Penicillin, Flavomycin, Avoparcin, Zinkbacitracin und Virginiamycin sind willkürlich so gewählt, daß in der Versuchsanordnung im Reihenverdünnungstest bei dezimaler Verdünnungsabstufung positive und negative Ergebnisse erhalten werden.

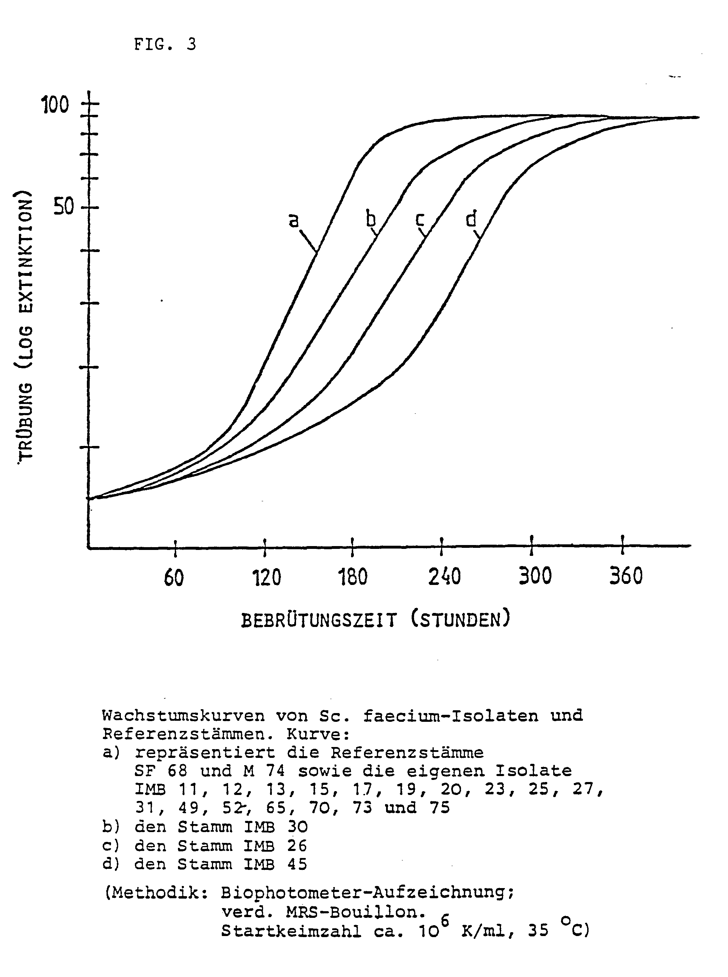

Zur Bestimmung der Wachstumsgeschwindigkeit, also der Generationszeit, wird nach Beimpfung eines flüssigen Nährmediums mit 1 % Impfsuspension (standardisierte Keimzahl) des jeweiligen Stammes der Anstieg der Trübung in Abhängigkeit von der Inkubationszeit in einem Biophotometer kontinuierlich aufgezeichnet und aus der mittels Koch scher Plattenmethode geeichten logarithmischen Phase die durchschnittliche Generationszeit der Stämme bei Optimalbedingungen kalkuliert (s. dazu Fig. 3). Die besten Stämme, nämlich SF 68, M 74, IMB 12, IMB 49 und IMB 52, weisen Generationszeiten von 18 bis 20 Minuten auf.

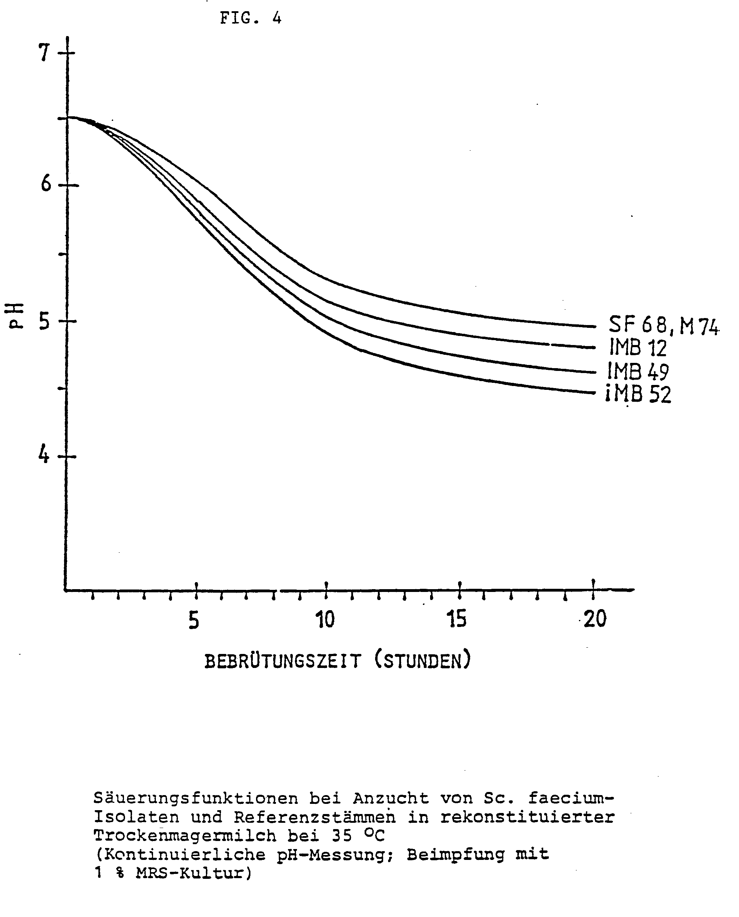

In Tabelle 4 sind die Ergebnisse der Säuerungsaktivitätsbestimmung aufgezeigt. Ausgehend von einem einheitlichen Ausgangs-pH-Wert von 6,52 in MRS-Bouillon und einer egalisierten Beimpfungs- und Bebrütungsform liefern die untersuchten Stämme sowie die Referenzstämme nach 24 Stunden unterschiedliche End-pH-Werte im Bereich von 5,2 bis 4,5. Der Stamm IMB 52 liegt dabei mit 4,55 am unteren Rand der Skala und ist somit als relativ stark säuerungsaktiv zu bezeichnen. Eine graphische Darstellung der pH-Funktionen einiger ausgewählter Stämme zeigt Fig. 4.

Die Ergebnisse der Hitzeresistenzprüfung sind aus Tabelle 5 ersichtlich. Als Testbedingungen sind willkürlich 15 Minuten bei 75 °C gewählt. Die Lebendkeimzahl, ausgedrückt als koloniebildende Einheiten (KbE) nach Koch'schem Plattenverfahren, wird unmittelbar vor und und nach der Erhitzung ohne spezifisch-kulturelle Wiederbelebungsschritte .auf MRS-Agar geprüft und die Keimreduktion als Vergleichsmaß errechnet. Die geringste Reduktion, d. h. die höchste relative Hitzeresistenz zeigt der Stamm IMB 23 mit einer Abnahme der Keimzahl unter den definierten Bedingungen von lediglich $3,4 \times 10^4$, die höchste erhitzungsbedingte Reduktion der Stamm IMB 31 mit $3,5 \times 10^6$. Der Stamm IMB 52 liegt bei diesem Prüfkriterium nach den Referenzstämmen an drittbester Stelle.

Bei Pilot-plant-Trocknungsversuchen von ausgewählten Sc. faecium-Stämmen und den Referenzstämmen werden die Stamme in Magermilchkonzentrat mit 30 % Trockensubstanz bei 35 °C während 20 bis 24 Stunden inkubiert. Am Ende der Bebrütungszeit liegt der pH-Wert, wie aus Vorversuchen erwartet, in Abhängigkeit von der Stammtype zwischen 4,5 und 5,2. Die erreichbaren Keimzahlwerte vor der Sprühtrocknung umfassen einen Bereich von $2,5 \times 10^5$ bis $2,7 \times 10^9$ (siehe dazu Tabelle 6). Der Stamm IMB 52 liegt dabei mit $2,5 \times 10^9$ an zweitbester Stelle inner-

halb der vorliegenden Testreihe hinsichtlich der Keimzahl, obwohl der pH-Wert im Konzentrat mit pH 4,7 verhältnismäßig niedrig ist.

Die Sprühtrocknung selbst hat auf Grund der schonenden Bedingungen nur bei einzelnen Stämmen erwähnenswerten Einfluß auf die Keimreduktion. In den meisten Fällen ist die Lebendkeimzahl vor der Sprühtrocknung im wesentlichen gleich jener nach der Sprühtrocknung, u. zw. unter Berücksichtigung eines Faktors von 3 bis 3,5 für die Aufkonzentrierung. Der Stamm IMB 52 hat dabei eine Keimzahlveränderung bei der Trocknung von $7,5 \times 10^9$ auf $5,5 \times 10^9$ aufzuweisen.

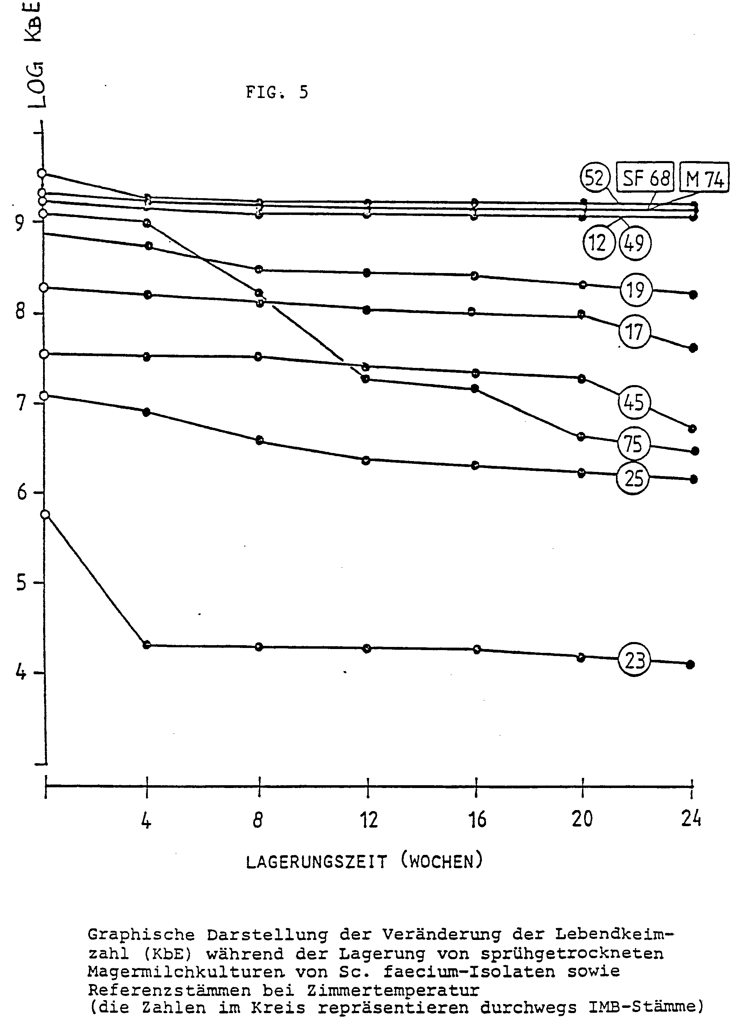

Ein essentielles Kriterium für einen als Futtermittelzusatz nutzbaren Keim ist vor allem dessen Stabilität in der trockenen Futtermittelmischung während der regulären Lagerzeit. Deshalb werden die auf Grund der beschriebenen Ergebnisse in die nähere Wahl gezogenen Sc. faecium-Stämme und die Referenzstämme bei Zimmertemperatur einem Lagerungsstabilitätstest unterworfen, der 6 Monate dauert. Dazu werden im Konzentrat vor der Beimpfung, nach der Beimpfung und in der Trockenmilch unmittelbar nach ihrer Herstellung die Keimzahlen als Basiswerte bestimmt. Danach wird eine Keimzahlbestimmung im Pulver in monatlichen Abständen mittels der Plattenmethode in üblicher Form vorgenommen (MRS-Medium, 72 Stunden bei 35 °C). Die Ergebnisse sind in Tabelle 7 zusammengefaßt und charakteristische Kurvenverläufe beispielhaft in Fig. 5 wiedergegeben. Neben den beiden Referenzstämmen SF 68 und M 74 zeichnen sich auch die eigenen Stämme IMB 49 und IMB 52 durch eine besonders gute Lagerungsstabilität aus.

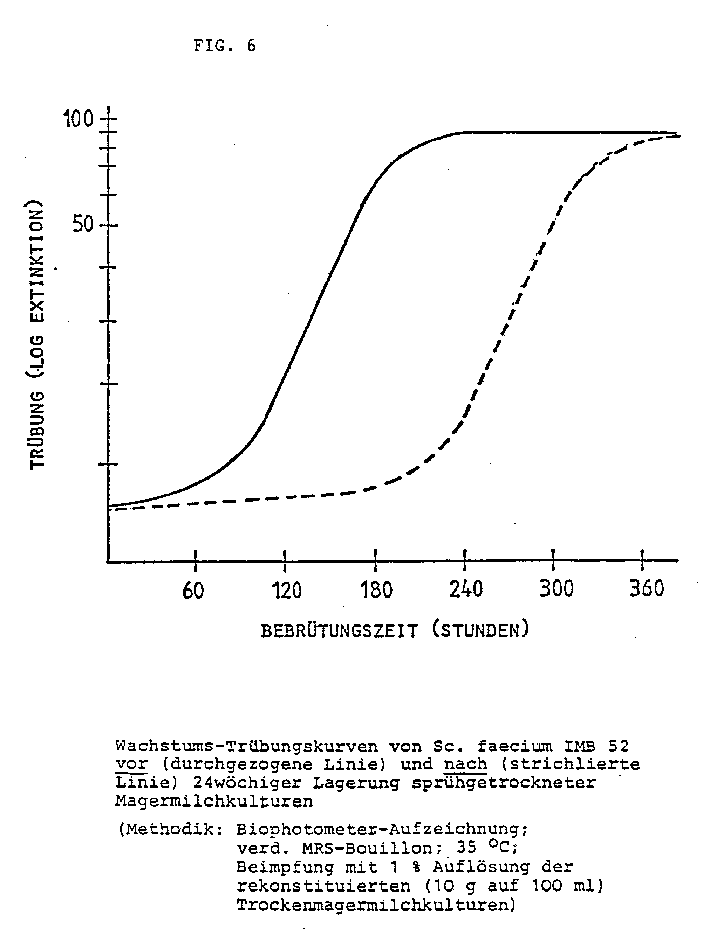

Die Prüfung des Anzuchtverhaltens von IMB 52 am Ende der sechsmonatigen Lagerzeit ergibt das in Fig. 6 dargestellte Resultat. Daraus ist ersichtlich, daß sich die sogenannte Zauderphase bei IMB 52 um etwa 2 Stunden verlängert, die anschließende logarithmische Phase jedoch erwartungsgemäß parallelen Verlauf im Vergleich zu frisch überimpften Kulturen aufweist.

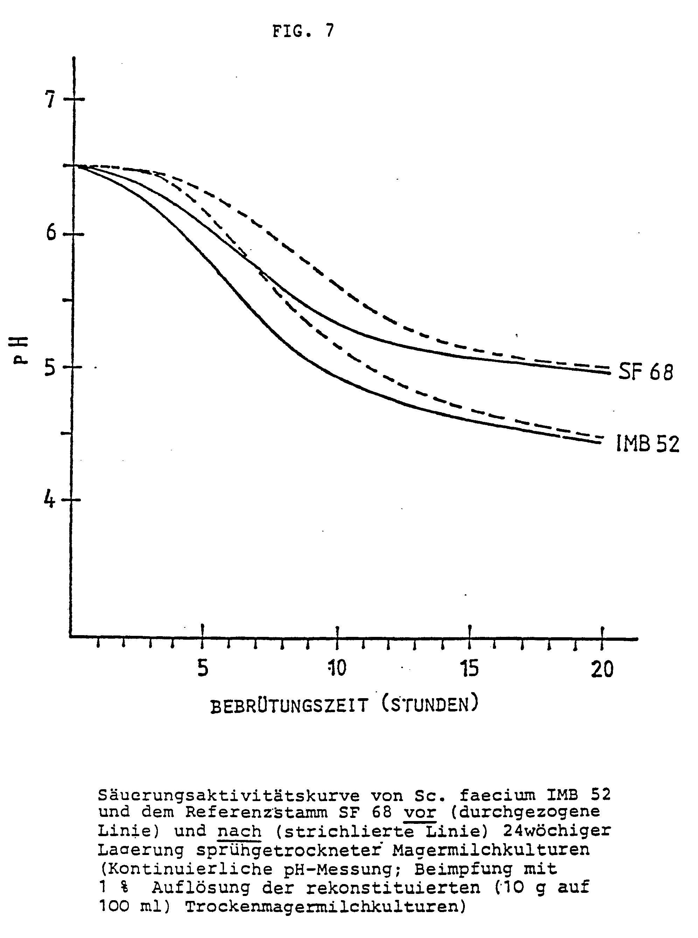

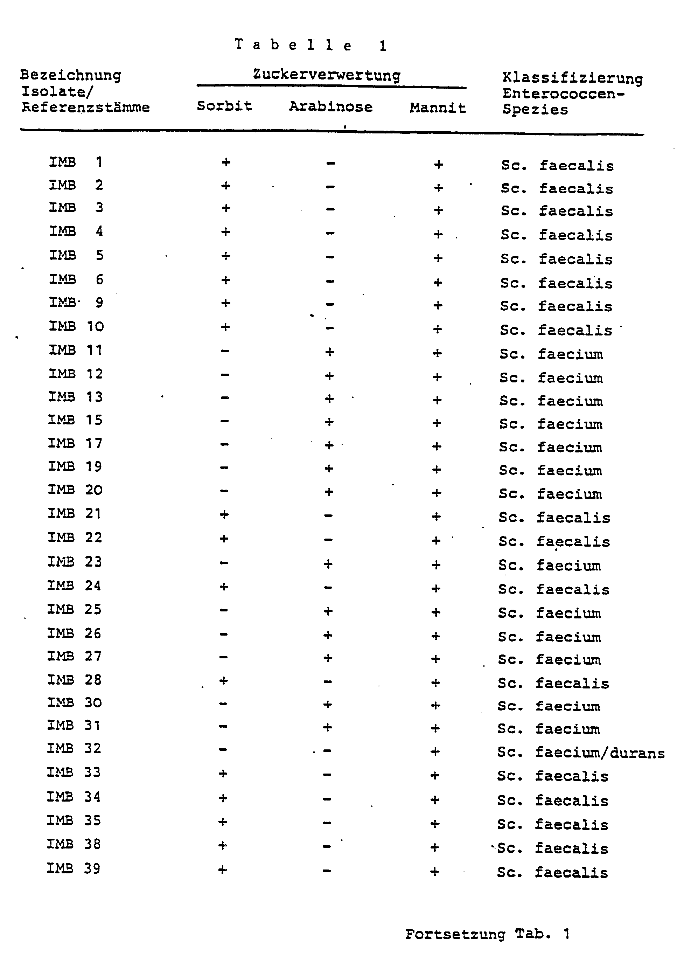

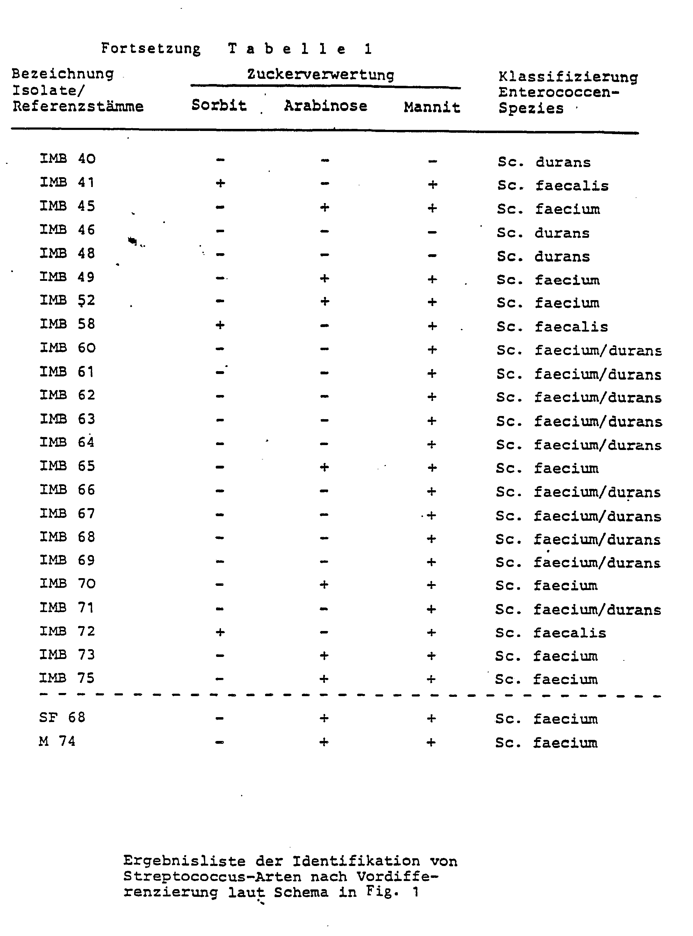

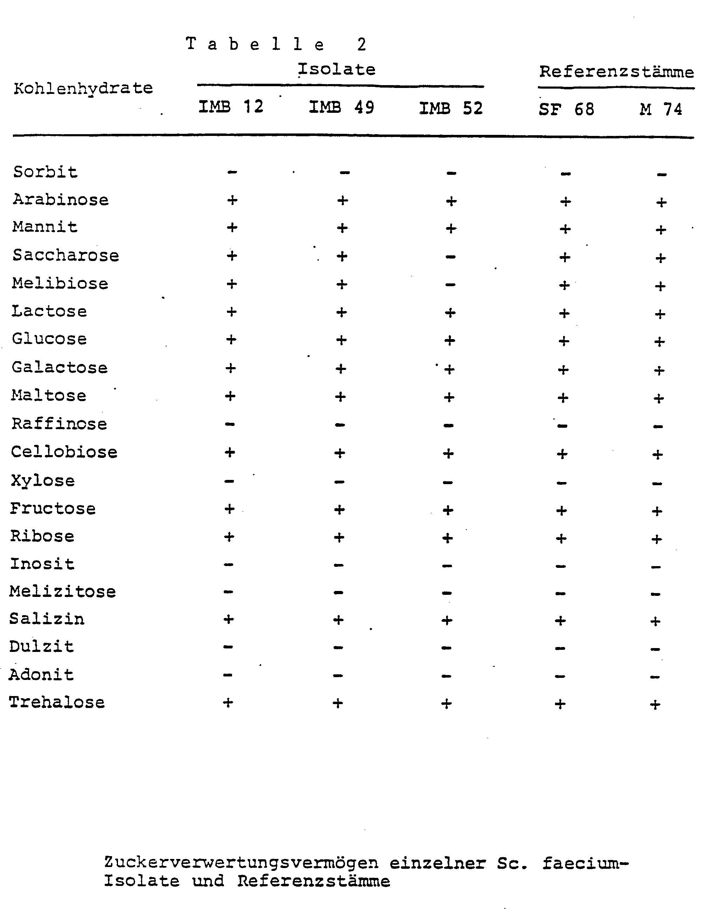

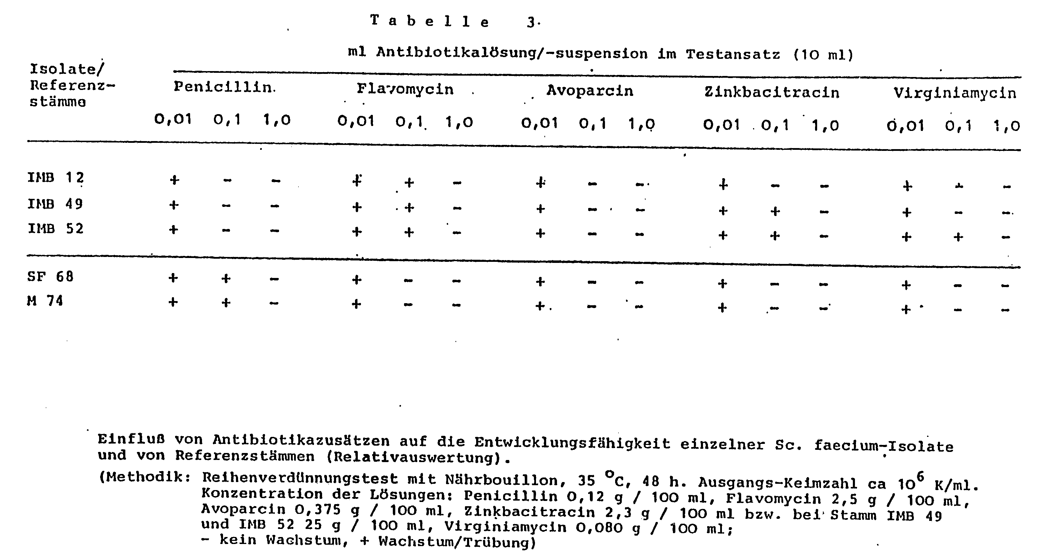

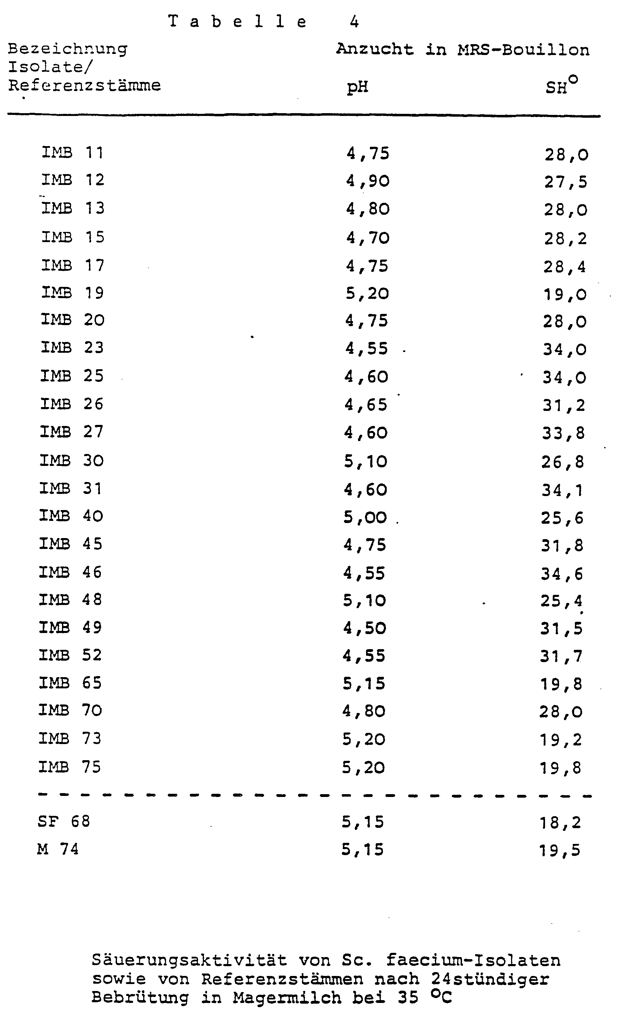

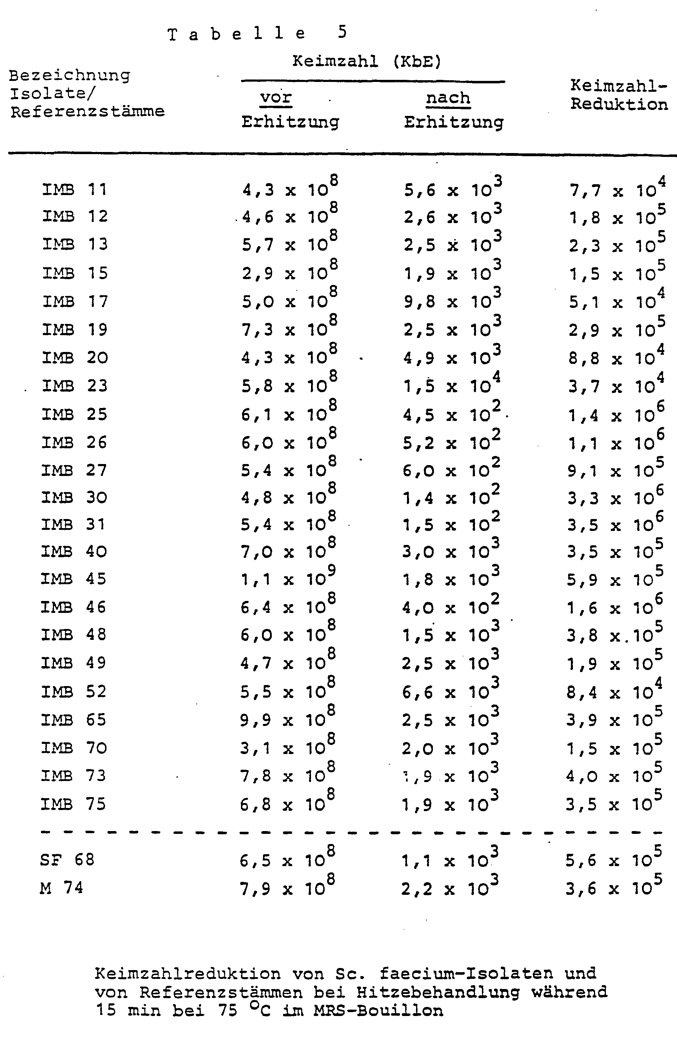

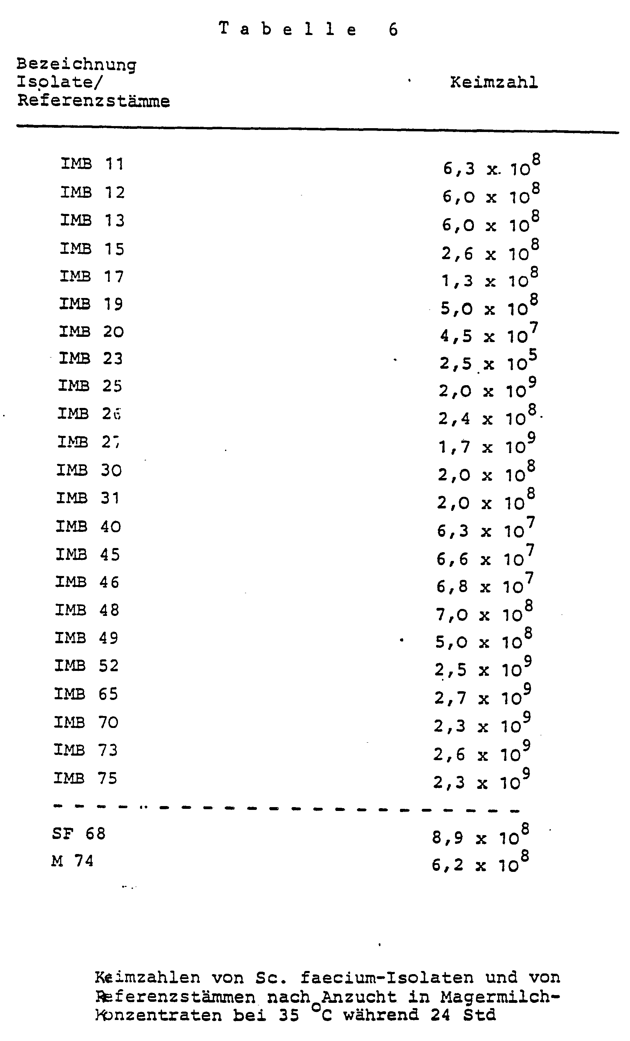

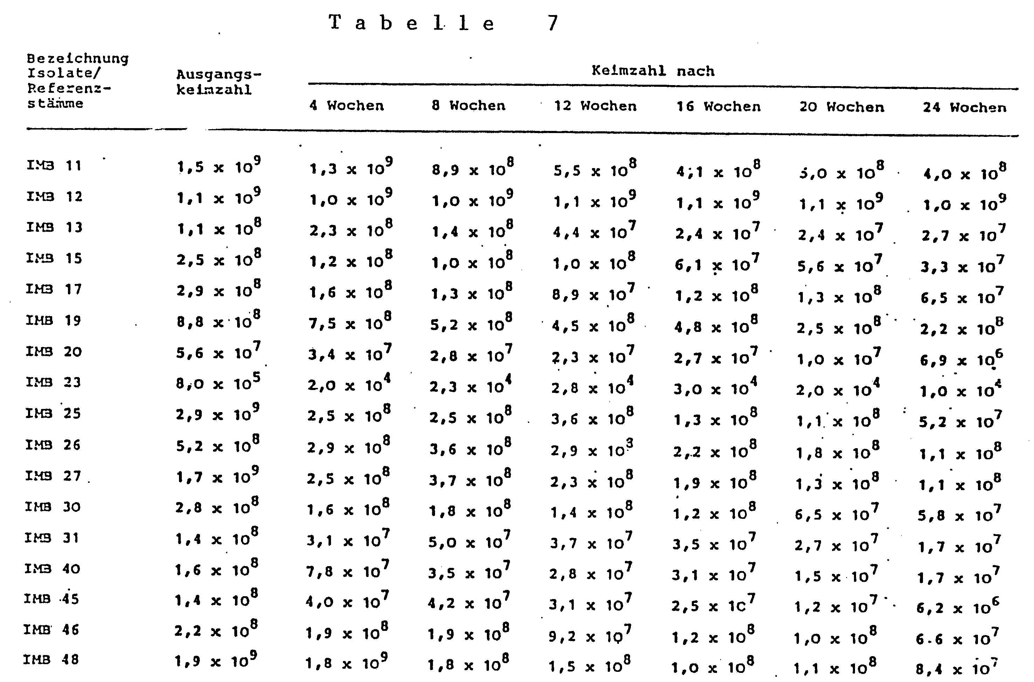

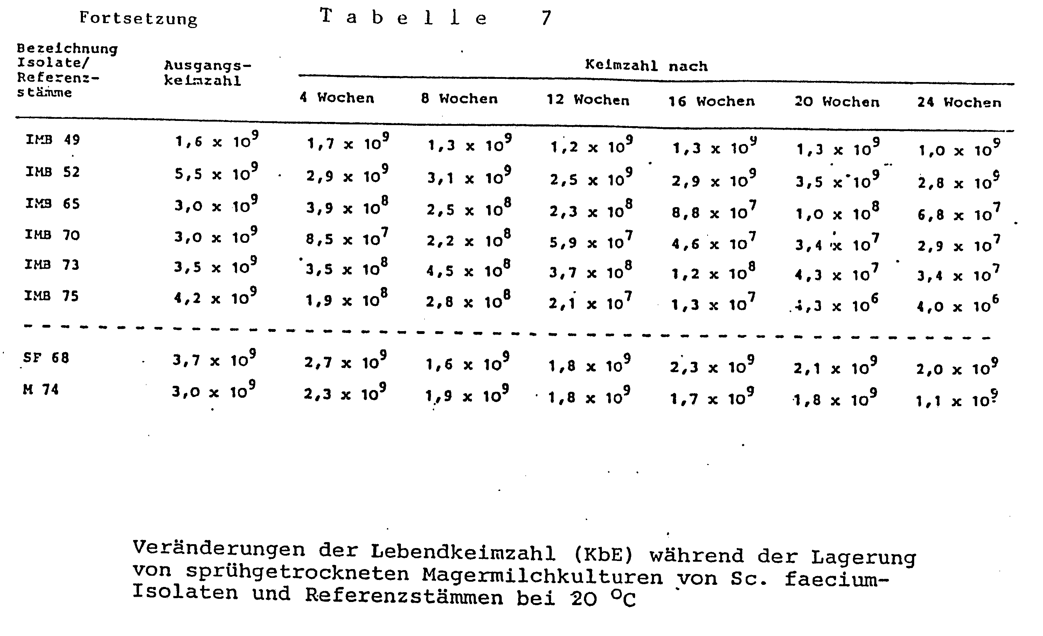

Auch bei der spezifischen Säuerungsaktivität sind sowohl bei Stamm IMB 52 als auch beim Referenzstamm SF 68 nach sechsmonatiger Lagerung der Kultur in trockenem Zustand Änderungen im Vergleich zu frisch überimpften Kulturen zu registrieren. Die Säuerungsgeschwindigkeit verzögert sich allerdings nur in den ersten Stunden des Beobachtungszeitraumes, in weiterer Folge wird die pH-Differenz aber wieder ausgeglichen und schließlich ein End-pH-Wert erreicht, der jenem frisch überimpfter Kulturen entspricht (siehe Fig. 7).

Für eine industrielle Züchtung des Mikroorganismus wird der Bakterienstamm in Magermilch gezüchtet, die vor Beimpfen auf 90 °C erhitzt und dann auf die Bebrütungstemperatur von 36 °C abgekühlt wird. Dann wird die Magermilch mit einer Impfmenge von 2 % mit der gezüchteten Kultur beimpft und etwa 22 bis 24 Stunden bis zum Erreichen eines pH-Wertes von 4,6 bebrütet. Dann wird durch Abkühlung das Bakterienwachstum und damit die Säuerung unterbrochen. Diese Kultur wird dann in eine auf etwa 30 % Trockensubstanz konzentrierte Magermilch eingeimpft, u. zw. wieder mit einer Impfmenge von 2 %. Das beimpfte Substrat wird dann bei 36 °C etwa 22 bis 24 Stunden bebrütet, bis wieder der pH-Wert auf 4,6 gesunken ist, wonach dann die Säuerung durch Kühlung unterbrochen wird. Während dieser Abkühlung wird das Substrat fortwährend gerührt. Das so erhaltene Magermilch-Bakterien-Konzentrat wird dann in einem herkömmlichen Trockenturm getrocknet, wobei die Zulufttemperatur etwa 150 °C und die Ablufttemperatur etwa 79 °C beträgt. Das so getrocknete Produkt hat dann einen Mindestkeimgehalt von $2,5 \times 10^9$ Keime pro Gramm. Die Haltbarkeit des Produktes ohne Veränderung der Keimfähigkeit beträgt dabei mindestens sechs Monate.

## Patentansprüche

1. Verwendung eines neuen Stammes von Streptococcus faecium Bakterien zur Absenkung des pH-Wertes im Magen-Darm-Trakt von Tieren, dadurch gekennzeichnet, daß Bakterien mit den identifizierenden Merkmalen des Stammes Streptococcus faecium IMB 52, hinterlegt unter DSM 3530, eingesetzt werden.

2. Verwendung nach Anspruch 1, dadurch gekennzeichnet, daß die Bakterien dem Futter unter Vorsäuerung desselben beigegeben werden.

3. Verwendung nach Anspruch 2, dadurch gekennzeichnet, daß die Bakterien dem Futter zur Erzeugung von Silagefutter mit hohem Säuerungsgrad beigegeben werden.

4. Verwendung von Bakterien mit den identifizierenden Merkmalen des Stamm, es Streptococcus faecium IMB 52 als mikrobieller Leistungsförderer.

5. Mikroorganismus insbesondere zur Regeneration der Darmflora von Tieren bzw. zur Absenkung des pH-Wertes im Magen-Darm-Trakt von Tieren bzw. zur Säuerung von pflanzlichen Nahrungs- und Futtermitteln, gekennzeichnet durch die identifizierenden Merkmale des Stammes Streptococcus faecium IMB 52, hinterlegt unter Nummer DSM 3530.

## Claims

1. Use of a new strain of Streptococcus faecium bacteria for reducing the pH value in the gastrointestinal tract of animals, characterised in that bacteria with the identifying features of the strain Streptococcus faecium IMB 52, deposited under DSM 3530, are used.

2. Use as claimed in claim 1, characterised in that the bacteria are added to the feed after having been pre-acidified.

3. Use as claimed in claim 2, characterised in that the bacteria are added to the feed to produce silage feed with a high degree of acidification.

4. Use of bacteria with the identifying features of the strain of Streptococcus faecium IMB 52 as a microbial performance booster.

5. Microorganism, particularly for regeneration of the intestinal flora of animals or to reduce the pH value in the gastro-intestinal tract of animals or for the acidification of vegetable nutritional and feed means, characterised by the identifying features of the strain Streptococcus faecium IMB 52, deposited under Number DMS 3530.

## Revendications

1. Utilisation d'une nouvelle souche de bactéries de Streptococcus faecium pour l'abaissement de la valeur du pH dans l'appareil gastro-intestinal d'animaux, caractérisée en ce que sont utilisées des bactéries avec les caractéristiques identifiées de la souche de Streptococcus faecium IMB 52, déposée sous la référence DSM 3530.

2. Utilisation selon la revendication 1, caractérisée en ce que les bactéries sont ajoutées à la nourriture, avec préacidification de celle-ci.

3. Utilisation selon la revendication 2, caractérisée en ce que les bactéries sont ajoutées à la nourriture, pour l'obtention d'une nourriture d'ensilage à haut degré d'acidité.

4. Utilisation de bactéries possédant les caractéristiques identifiées de la souche de Streptococcus faecium IMB 52, comme vecteur d'activité microbienne.

5. Micro-organisme, en particulier pour la régénération de la flore intestinale d'animaux, par abaissement de la valeur du pH dans l'appareil gastro-intestinal, par acidification du fourrage et de la nourriture, caractérisé par les propriétés identifiées de la souche Streptococcus faecium IMB 52, déposée sous la référence DSM 3530.

*Probe*

↓

*Keimzahl n. Koch*
*CATC-Agarplatten, 45 °C, 36 Stunden*

↓

*Anreicherung*
*KAA-Brühe, 37 °C, 24 Stunden*

FIG. 1

↓

*fraktionierter Ausstrich*
*CATC-Agar, 37 °C, 3 Tage*

↓

*Glucose-Tryptose-Brühe, 10 °C, 6 Tage*

⊕        ⊖

*6,5 % NaCl*
*Glucose-Tryptose-Bouillon*
*37 °C, 2 Tage*

*nach 37 °C, 2 Tagen*

⊖    ⊕

⊕

*Konfirmativtest*
*Gluc.Trypt.B.*
*pH 9,6, 37 °C, 2 Tge.*

*Sorbitbouillon*
*37 °C, 2 Tage*

*Stärkeagar, 37 °C, 4 Tage*
*Hydrolyse*

⊕    ⊖

⊕      ⊖

⊕

*Sc. bovis*     *Sc. thermophilus*

*Sc. uberis*

| *Sc. faecium* |
| *(Sc. durans)* |

*Azid-Blutagar*
*37 °C, 3 Tage*
*ß-Hämolyse*

⊕    ⊖

*Sc. faecalis*
*var. zymogenes*
*(oder hämolyticus)*

*Gelatinestichkultur*
*25 °C, 10 Tage*
*Verflüssigung*

⊕    ⊖

*Sc. faecalis*
*var. liquefaciens*

*Sc. faecalis*
*var. faecalis*

Schematische Darstellung des Isolations- bzw.
Identifikationsganges für Sc. faecium/durans
(CATC = Citrat-Azid-Tween-Carbonat-Agar,
KAA= Kanamycin-Äsculin-Azid-Agar)

FIG. 2

Elektrophoretisches Trennbild intrazellulärer
Proteine von Sc. faecium-Isolaten und Referenzstämmen. (Polyacrylamidgel, pH 8,9,
Trennzeit 6 Stunden bei 18 °C und 400 Volt;
Gefärbt mit Amidoschwarz/Coomassie )

FIG. 3

Wachstumskurven von Sc. faecium-Isolaten und
Referenzstämmen. Kurve:
a) repräsentiert die Referenzstämme
   SF 68 und M 74 sowie die eigenen Isolate
   IMB 11, 12, 13, 15, 17, 19, 20, 23, 25, 27,
   31, 49, 52, 65, 70, 73 und 75
b) den Stamm IMB 30
c) den Stamm IMB 26
d) den Stamm IMB 45

(Methodik: Biophotometer-Aufzeichnung;
         verd. MRS-Bouillon.
         Startkeimzahl ca. $10^6$ K/ml, 35 °C)

FIG. 4

Säuerungsfunktionen bei Anzucht von Sc. faecium-
Isolaten und Referenzstämmen in rekonstituierter
Trockenmagermilch bei 35 °C
(Kontinuierliche pH-Messung; Beimpfung mit
1 % MRS-Kultur)

FIG. 5

Graphische Darstellung der Veränderung der Lebendkeimzahl (KbE) während der Lagerung von sprühgetrockneten
Magermilchkulturen von Sc. faecium-Isolaten sowie
Referenzstämmen bei Zimmertemperatur
(die Zahlen im Kreis repräsentieren durchwegs IMB-Stämme)

FIG. 6

Wachstums-Trübungskurven von Sc. faecium IMB 52
vor (durchgezogene Linie) und nach (strichlierte
Linie) 24wöchiger Lagerung sprühgetrockneter
Magermilchkulturen

(Methodik: Biophotometer-Aufzeichnung;
verd. MRS-Bouillon; 35 °C;
Beimpfung mit 1 % Auflösung der
rekonstituierten (10 g auf 100 ml)
Trockenmagermilchkulturen)

FIG. 7

Säuerungsaktivitätskurve von Sc. faecium IMB 52
und dem Referenzstamm SF 68 vor (durchgezogene
Linie) und nach (strichlierte Linie) 24wöchiger
Lagerung sprühgetrockneter Magermilchkulturen
(Kontinuierliche pH-Messung; Beimpfung mit
1 % Auflösung der rekonstituierten (10 g auf
100 ml) Trockenmagermilchkulturen)

Tabelle 1

| Bezeichnung Isolate/ Referenzstämme | Zuckerverwertung | | | Klassifizierung Enterococcen- Spezies |
|---|---|---|---|---|
| | Sorbit | Arabinose | Mannit | |
| IMB 1 | + | - | + | Sc. faecalis |
| IMB 2 | + | - | + | Sc. faecalis |
| IMB 3 | + | - | + | Sc. faecalis |
| IMB 4 | + | - | + | Sc. faecalis |
| IMB 5 | + | - | + | Sc. faecalis |
| IMB 6 | + | - | + | Sc. faecalis |
| IMB 9 | + | - | + | Sc. faecalis |
| IMB 10 | + | - | + | Sc. faecalis |
| IMB 11 | - | + | + | Sc. faecium |
| IMB 12 | - | + | + | Sc. faecium |
| IMB 13 | - | + | + | Sc. faecium |
| IMB 15 | - | + | + | Sc. faecium |
| IMB 17 | - | + | + | Sc. faecium |
| IMB 19 | - | + | + | Sc. faecium |
| IMB 20 | - | + | + | Sc. faecium |
| IMB 21 | + | - | + | Sc. faecalis |
| IMB 22 | + | - | + | Sc. faecalis |
| IMB 23 | - | + | + | Sc. faecium |
| IMB 24 | + | - | + | Sc. faecalis |
| IMB 25 | - | + | + | Sc. faecium |
| IMB 26 | - | + | + | Sc. faecium |
| IMB 27 | - | + | + | Sc. faecium |
| IMB 28 | + | - | + | Sc. faecalis |
| IMB 30 | - | + | + | Sc. faecium |
| IMB 31 | - | + | + | Sc. faecium |
| IMB 32 | - | - | + | Sc. faecium/durans |
| IMB 33 | + | - | + | Sc. faecalis |
| IMB 34 | + | - | + | Sc. faecalis |
| IMB 35 | + | - | + | Sc. faecalis |
| IMB 38 | + | - | + | Sc. faecalis |
| IMB 39 | + | - | + | Sc. faecalis |

Fortsetzung  T a b e l l e  1

| Bezeichnung Isolate/ Referenzstämme | Zuckerverwertung | | | Klassifizierung Enterococcen- Spezies |
|---|---|---|---|---|
| | Sorbit | Arabinose | Mannit | |
| IMB 40 | − | − | − | Sc. durans |
| IMB 41 | + | − | + | Sc. faecalis |
| IMB 45 | − | + | + | Sc. faecium |
| IMB 46 | − | − | − | Sc. durans |
| IMB 48 | − | − | − | Sc. durans |
| IMB 49 | − | + | + | Sc. faecium |
| IMB 52 | − | + | + | Sc. faecium |
| IMB 58 | + | − | + | Sc. faecalis |
| IMB 60 | − | − | + | Sc. faecium/durans |
| IMB 61 | − | − | + | Sc. faecium/durans |
| IMB 62 | − | − | + | Sc. faecium/durans |
| IMB 63 | − | − | + | Sc. faecium/durans |
| IMB 64 | − | − | + | Sc. faecium/durans |
| IMB 65 | − | + | + | Sc. faecium |
| IMB 66 | − | − | + | Sc. faecium/durans |
| IMB 67 | − | − | + | Sc. faecium/durans |
| IMB 68 | − | − | + | Sc. faecium/durans |
| IMB 69 | − | − | + | Sc. faecium/durans |
| IMB 70 | − | + | + | Sc. faecium |
| IMB 71 | − | − | + | Sc. faecium/durans |
| IMB 72 | + | − | + | Sc. faecalis |
| IMB 73 | − | + | + | Sc. faecium |
| IMB 75 | − | + | + | Sc. faecium |
| SF 68 | − | + | + | Sc. faecium |
| M 74 | − | + | + | Sc. faecium |

Ergebnisliste der Identifikation von
Streptococcus-Arten nach Vordifferenzierung laut Schema in Fig. 1

T a b e l l e   2

| Kohlenhydrate | Isolate | | | Referenzstämme | |
|---|---|---|---|---|---|
| | IMB 12 | IMB 49 | IMB 52 | SF 68 | M 74 |
| Sorbit | − | − | − | − | − |
| Arabinose | + | + | + | + | + |
| Mannit | + | + | + | + | + |
| Saccharose | + | + | − | + | + |
| Melibiose | + | + | − | + | + |
| Lactose | + | + | + | + | + |
| Glucose | + | + | + | + | + |
| Galactose | + | + | + | + | + |
| Maltose | + | + | + | + | + |
| Raffinose | − | − | − | − | − |
| Cellobiose | + | + | + | + | + |
| Xylose | − | − | − | − | − |
| Fructose | + | + | + | + | + |
| Ribose | + | + | + | + | + |
| Inosit | − | − | − | − | − |
| Melizitose | − | − | − | − | − |
| Salizin | + | + | + | + | + |
| Dulzit | − | − | − | − | − |
| Adonit | − | − | − | − | − |
| Trehalose | + | + | + | + | + |

Zuckerverwertungsvermögen einzelner Sc. faecium-
Isolate und Referenzstämme

EP 0 219 488 B1

T a b e l l e    3.

### ml Antibiotikalösung/-suspension im Testansatz (10 ml)

| Isolate/ Referenz-stämme | Penicillin | | | Flavomycin | | | Avoparcin | | | Zinkbacitracin | | | Virginiamycin | | |
|---|---|---|---|---|---|---|---|---|---|---|---|---|---|---|---|
| | 0,01 | 0,1 | 1,0 | 0,01 | 0,1 | 1,0 | 0,01 | 0,1 | 1,0 | 0,01 | 0,1 | 1,0 | 0,01 | 0,1 | 1,0 |
| IMB 12 | + | − | − | + | + | − | + | − | − | + | − | − | + | − | − |
| IMB 49 | + | − | − | + | + | − | + | − | − | + | + | − | + | − | − |
| IMB 52 | + | − | − | + | + | − | + | − | − | + | + | − | + | + | − |
| SF 68 | + | + | − | + | − | − | + | − | − | + | − | − | + | − | − |
| M 74 | + | + | − | + | − | − | + | − | − | + | − | − | + | − | − |

Einfluß von Antibiotikazusätzen auf die Entwicklungsfähigkeit einzelner Sc. faecium-Isolate und von Referenzstämmen (Relativauswertung).

(Methodik: Reihenverdünnungstest mit Nährbouillon, 35 °C, 48 h. Ausgangs-Keimzahl ca $10^6$ K/ml. Konzentration der Lösungen: Penicillin 0,12 g / 100 ml, Flavomycin 2,5 g / 100 ml, Avoparcin 0,375 g / 100 ml, Zinkbacitracin 2,3 g / 100 ml bzw. bei Stamm IMB 49 und IMB 52 25 g / 100 ml, Virginiamycin 0,080 g / 100 ml;
− kein Wachstum, + Wachstum/Trübung)

T a b e l l e  4

| Bezeichnung Isolate/ Referenzstämme | Anzucht in MRS-Bouillon | |
|---|---|---|
| | pH | SH$^{\circ}$ |
| IMB 11 | 4,75 | 28,0 |
| IMB 12 | 4,90 | 27,5 |
| IMB 13 | 4,80 | 28,0 |
| IMB 15 | 4,70 | 28,2 |
| IMB 17 | 4,75 | 28,4 |
| IMB 19 | 5,20 | 19,0 |
| IMB 20 | 4,75 | 28,0 |
| IMB 23 | 4,55 | 34,0 |
| IMB 25 | 4,60 | 34,0 |
| IMB 26 | 4,65 | 31,2 |
| IMB 27 | 4,60 | 33,8 |
| IMB 30 | 5,10 | 26,8 |
| IMB 31 | 4,60 | 34,1 |
| IMB 40 | 5,00 | 25,6 |
| IMB 45 | 4,75 | 31,8 |
| IMB 46 | 4,55 | 34,6 |
| IMB 48 | 5,10 | 25,4 |
| IMB 49 | 4,50 | 31,5 |
| IMB 52 | 4,55 | 31,7 |
| IMB 65 | 5,15 | 19,8 |
| IMB 70 | 4,80 | 28,0 |
| IMB 73 | 5,20 | 19,2 |
| IMB 75 | 5,20 | 19,8 |
| SF 68 | 5,15 | 18,2 |
| M 74 | 5,15 | 19,5 |

Säuerungsaktivität von Sc. faecium-Isolaten sowie von Referenzstämmen nach 24stündiger Bebrütung in Magermilch bei 35 °C

T a b e l l e   5

| Bezeichnung Isolate/ Referenzstämme | Keimzahl (KbE) | | Keimzahl- Reduktion |
|---|---|---|---|
| | vor Erhitzung | nach Erhitzung | |
| IMB 11 | $4,3 \times 10^8$ | $5,6 \times 10^3$ | $7,7 \times 10^4$ |
| IMB 12 | $4,6 \times 10^8$ | $2,6 \times 10^3$ | $1,8 \times 10^5$ |
| IMB 13 | $5,7 \times 10^8$ | $2,5 \times 10^3$ | $2,3 \times 10^5$ |
| IMB 15 | $2,9 \times 10^8$ | $1,9 \times 10^3$ | $1,5 \times 10^5$ |
| IMB 17 | $5,0 \times 10^8$ | $9,8 \times 10^3$ | $5,1 \times 10^4$ |
| IMB 19 | $7,3 \times 10^8$ | $2,5 \times 10^3$ | $2,9 \times 10^5$ |
| IMB 20 | $4,3 \times 10^8$ | $4,9 \times 10^3$ | $8,8 \times 10^4$ |
| IMB 23 | $5,8 \times 10^8$ | $1,5 \times 10^4$ | $3,7 \times 10^4$ |
| IMB 25 | $6,1 \times 10^8$ | $4,5 \times 10^2$ | $1,4 \times 10^6$ |
| IMB 26 | $6,0 \times 10^8$ | $5,2 \times 10^2$ | $1,1 \times 10^6$ |
| IMB 27 | $5,4 \times 10^8$ | $6,0 \times 10^2$ | $9,1 \times 10^5$ |
| IMB 30 | $4,8 \times 10^8$ | $1,4 \times 10^2$ | $3,3 \times 10^6$ |
| IMB 31 | $5,4 \times 10^8$ | $1,5 \times 10^2$ | $3,5 \times 10^6$ |
| IMB 40 | $7,0 \times 10^8$ | $3,0 \times 10^3$ | $3,5 \times 10^5$ |
| IMB 45 | $1,1 \times 10^9$ | $1,8 \times 10^3$ | $5,9 \times 10^5$ |
| IMB 46 | $6,4 \times 10^8$ | $4,0 \times 10^2$ | $1,6 \times 10^6$ |
| IMB 48 | $6,0 \times 10^8$ | $1,5 \times 10^3$ | $3,8 \times 10^5$ |
| IMB 49 | $4,7 \times 10^8$ | $2,5 \times 10^3$ | $1,9 \times 10^5$ |
| IMB 52 | $5,5 \times 10^8$ | $6,6 \times 10^3$ | $8,4 \times 10^4$ |
| IMB 65 | $9,9 \times 10^8$ | $2,5 \times 10^3$ | $3,9 \times 10^5$ |
| IMB 70 | $3,1 \times 10^8$ | $2,0 \times 10^3$ | $1,5 \times 10^5$ |
| IMB 73 | $7,8 \times 10^8$ | $1,9 \times 10^3$ | $4,0 \times 10^5$ |
| IMB 75 | $6,8 \times 10^8$ | $1,9 \times 10^3$ | $3,5 \times 10^5$ |
| SF 68 | $6,5 \times 10^8$ | $1,1 \times 10^3$ | $5,6 \times 10^5$ |
| M 74 | $7,9 \times 10^8$ | $2,2 \times 10^3$ | $3,6 \times 10^5$ |

Keimzahlreduktion von Sc. faecium-Isolaten und von Referenzstämmen bei Hitzebehandlung während 15 min bei 75 $^{\circ}$C im MRS-Bouillon

Tabelle 6

| Bezeichnung Isolate/ Referenzstämme | Keimzahl |
|---|---|
| IMB 11 | $6,3 \times 10^8$ |
| IMB 12 | $6,0 \times 10^8$ |
| IMB 13 | $6,0 \times 10^8$ |
| IMB 15 | $2,6 \times 10^8$ |
| IMB 17 | $1,3 \times 10^8$ |
| IMB 19 | $5,0 \times 10^8$ |
| IMB 20 | $4,5 \times 10^7$ |
| IMB 23 | $2,5 \times 10^5$ |
| IMB 25 | $2,0 \times 10^9$ |
| IMB 26 | $2,4 \times 10^8$ |
| IMB 27 | $1,7 \times 10^9$ |
| IMB 30 | $2,0 \times 10^8$ |
| IMB 31 | $2,0 \times 10^8$ |
| IMB 40 | $6,3 \times 10^7$ |
| IMB 45 | $6,6 \times 10^7$ |
| IMB 46 | $6,8 \times 10^7$ |
| IMB 48 | $7,0 \times 10^8$ |
| IMB 49 | $5,0 \times 10^8$ |
| IMB 52 | $2,5 \times 10^9$ |
| IMB 65 | $2,7 \times 10^9$ |
| IMB 70 | $2,3 \times 10^9$ |
| IMB 73 | $2,6 \times 10^9$ |
| IMB 75 | $2,3 \times 10^9$ |
| SF 68 | $8,9 \times 10^8$ |
| M 74 | $6,2 \times 10^8$ |

Keimzahlen von Sc. faecium-Isolaten und von Referenzstämmen nach Anzucht in Magermilch-Konzentraten bei 35 °C während 24 Std

14

| Bezeichnung Isolate/ Referenz- stämme | Ausgangs- keimzahl | Keimzahl nach | | | | | |
|---|---|---|---|---|---|---|---|
| | | 4 Wochen | 8 Wochen | 12 Wochen | 16 Wochen | 20 Wochen | 24 Wochen |
| IMB 11 | $1,5 \times 10^9$ | $1,3 \times 10^9$ | $8,9 \times 10^8$ | $5,5 \times 10^8$ | $4;1 \times 10^8$ | $5,0 \times 10^8$ | $4,0 \times 10^8$ |
| IMB 12 | $1,1 \times 10^9$ | $1,0 \times 10^9$ | $1,0 \times 10^9$ | $1,1 \times 10^9$ | $1,1 \times 10^9$ | $1,1 \times 10^9$ | $1,0 \times 10^9$ |
| IMB 13 | $1,1 \times 10^8$ | $2,3 \times 10^8$ | $1,4 \times 10^8$ | $4,4 \times 10^7$ | $2,4 \times 10^7$ | $2,4 \times 10^7$ | $2,7 \times 10^7$ |
| IMB 15 | $2,5 \times 10^8$ | $1,2 \times 10^8$ | $1,0 \times 10^8$ | $1,0 \times 10^8$ | $6,1 \times 10^7$ | $5,6 \times 10^7$ | $3,3 \times 10^7$ |
| IMB 17 | $2,9 \times 10^8$ | $1,6 \times 10^8$ | $1,3 \times 10^8$ | $8,9 \times 10^7$ | $1,2 \times 10^8$ | $1,3 \times 10^8$ | $6,5 \times 10^7$ |
| IMB 19 | $8,8 \times 10^8$ | $7,5 \times 10^8$ | $5,2 \times 10^8$ | $4,5 \times 10^8$ | $4,8 \times 10^8$ | $2,5 \times 10^8$ | $2,2 \times 10^8$ |
| IMB 20 | $5,6 \times 10^7$ | $3,4 \times 10^7$ | $2,8 \times 10^7$ | $2,3 \times 10^7$ | $2,7 \times 10^7$ | $1,0 \times 10^7$ | $6,9 \times 10^6$ |
| IMB 23 | $8,0 \times 10^5$ | $2,0 \times 10^4$ | $2,3 \times 10^4$ | $2,8 \times 10^4$ | $3,0 \times 10^4$ | $2,0 \times 10^4$ | $1,0 \times 10^4$ |
| IMB 25 | $2,9 \times 10^9$ | $2,5 \times 10^8$ | $2,5 \times 10^8$ | $3,6 \times 10^8$ | $1,3 \times 10^8$ | $1,1 \times 10^8$ | $5,2 \times 10^7$ |
| IMB 26 | $5,2 \times 10^8$ | $2,9 \times 10^8$ | $3,6 \times 10^8$ | $2,9 \times 10^3$ | $2,2 \times 10^8$ | $1,8 \times 10^8$ | $1,1 \times 10^8$ |
| IMB 27 | $1,7 \times 10^9$ | $2,5 \times 10^8$ | $3,7 \times 10^8$ | $2,3 \times 10^8$ | $1,9 \times 10^8$ | $1,3 \times 10^8$ | $1,1 \times 10^8$ |
| IMB 30 | $2,8 \times 10^8$ | $1,6 \times 10^8$ | $1,8 \times 10^8$ | $1,4 \times 10^8$ | $1,2 \times 10^8$ | $6,5 \times 10^7$ | $5,8 \times 10^7$ |
| IMB 31 | $1,4 \times 10^8$ | $3,1 \times 10^7$ | $5,0 \times 10^7$ | $3,7 \times 10^7$ | $3,5 \times 10^7$ | $2,7 \times 10^7$ | $1,7 \times 10^7$ |
| IMB 40 | $1,6 \times 10^8$ | $7,8 \times 10^7$ | $3,5 \times 10^7$ | $2,8 \times 10^7$ | $3,1 \times 10^7$ | $1,5 \times 10^7$ | $1,7 \times 10^7$ |
| IMB 45 | $1,4 \times 10^8$ | $4,0 \times 10^7$ | $4,2 \times 10^7$ | $3,1 \times 10^7$ | $2,5 \times 1c^7$ | $1,2 \times 10^7$ | $6,2 \times 10^6$ |
| IMB 46 | $2,2 \times 10^8$ | $1,9 \times 10^8$ | $1,9 \times 10^8$ | $9,2 \times 10^7$ | $1,2 \times 10^8$ | $1,0 \times 10^8$ | $6.6 \times 10^7$ |
| IMB 48 | $1,9 \times 10^9$ | $1,8 \times 10^9$ | $1,8 \times 10^8$ | $1,5 \times 10^8$ | $1,0 \times 10^8$ | $1,1 \times 10^8$ | $8,4 \times 10^7$ |

EP 0 219 488 B1

# Tabelle 7

| Bezeichnung Isolate/ Referenz- stämme | Ausgangs- keimzahl | Keimzahl nach | | | | | |
|---|---|---|---|---|---|---|---|
| | | 4 Wochen | 8 Wochen | 12 Wochen | 16 Wochen | 20 Wochen | 24 Wochen |
| IMB 49 | $1,6 \times 10^9$ | $1,7 \times 10^9$ | $1,3 \times 10^9$ | $1,2 \times 10^9$ | $1,3 \times 10^9$ | $1,3 \times 10^9$ | $1,0 \times 10^9$ |
| IMB 52 | $5,5 \times 10^9$ | $2,9 \times 10^9$ | $3,1 \times 10^9$ | $2,5 \times 10^9$ | $2,9 \times 10^9$ | $3,5 \times 10^9$ | $2,8 \times 10^9$ |
| IMB 65 | $3,0 \times 10^9$ | $3,9 \times 10^8$ | $2,5 \times 10^8$ | $2,3 \times 10^8$ | $8,8 \times 10^7$ | $1,0 \times 10^8$ | $6,8 \times 10^7$ |
| IMB 70 | $3,0 \times 10^9$ | $8,5 \times 10^7$ | $2,2 \times 10^8$ | $5,9 \times 10^7$ | $4,6 \times 10^7$ | $3,4 \times 10^7$ | $2,9 \times 10^7$ |
| IMB 73 | $3,5 \times 10^9$ | $3,5 \times 10^8$ | $4,5 \times 10^8$ | $3,7 \times 10^8$ | $1,2 \times 10^8$ | $4,3 \times 10^7$ | $3,4 \times 10^7$ |
| IMB 75 | $4,2 \times 10^9$ | $1,9 \times 10^8$ | $2,8 \times 10^8$ | $2,1 \times 10^7$ | $1,3 \times 10^7$ | $4,3 \times 10^6$ | $4,0 \times 10^6$ |
| SF 68 | $3,7 \times 10^9$ | $2,7 \times 10^9$ | $1,6 \times 10^9$ | $1,8 \times 10^9$ | $2,3 \times 10^9$ | $2,1 \times 10^9$ | $2,0 \times 10^9$ |
| M 74 | $3,0 \times 10^9$ | $2,3 \times 10^9$ | $1,9 \times 10^9$ | $1,8 \times 10^9$ | $1,7 \times 10^9$ | $1,8 \times 10^9$ | $1,1 \times 10^9$ |

Veränderungen der Lebendkeimzahl (KbE) während der Lagerung von sprühgetrockneten Magermilchkulturen von Sc. faecium-Isolaten und Referenzstämmen bei 20 °C